# EUROPÄISCHE PATENTANMELDUNG

(11) **EP 1 424 099 A1**
(43) Veröffentlichungstag der Anmeldung: **02.06.2004**
(21) Anmeldenummer: 03023239.1
(22) Anmeldetag: 14.10.2003
(51) Int. Cl.: A61N 2/02

(54) **Einrichtung und Verfahren zur Regenerierung von biologischem Gewebe**

(30) Priorität: 28.11.2002 DE 10255791
(71) Anmelder: Bühler, Urs, 9240 Uzwil (CH)
(72) Erfinder: Bühler, Urs, 9240 Uzwil (CH); Bärtschi-Herrera, Paloma, 8617 Mönchaltdorf ZH (CH)
(74) Vertreter: Frommhold, Joachim, Dr.

(57) **Zusammenfassung**

Die Erfindung betrifft eine Einrichtung sowie ein Verfahren zur Regenerierung von biologischem Gewebe, insbesondere der verschiedenen biologischen Gewebe von Tier oder Mensch, wie zum Beispiel Muskeln, Nerven, Knorpel oder Sehnen.

Die Einrichtung besteht aus mindestens einem Empfänger (50) und einer zugehörigen Speicher- und Auswerteeinheit für Bioinformationen, einem Verstärker (60) für diese Bioinformationen und einem Sender (80) zur Einspeisung der verstärkten bzw. verdichteten Bioinformation in das zu regenerierende Gewebe.

## Beschreibung

Die Erfindung betrifft eine Einrichtung zur Regenerierung von biologischem Gewebe von Mensch und Tier oder auch von Vögeln, wie zum Beispiel Muskel- oder Nervenfasern, Sehnen. Die Erfindung betrifft weiterhin ein Verfahren zur Regenerierung von solchen biologischen Geweben.

Unterstützende Mittel zur Heilung bzw. Regenerierung von biologischen Geweben sind hinlänglich bekannt. So offenbaren die DD 203229 und DD 226773 Handinstrumente unter Nutzung von Ultraschallfestkörperschwingungen, um solche Gewebe zu schneiden oder auch zur Entfernung von Zahnstein. Hierbei schwingt das Instrument als Halbwellenresonatorin seiner Resonanzfrequenz. Das Arbeitssegment mit einem konvex gekrümmten Rücken ist auf einem zylindrischen Schaftsegment, mit einem, sich nach einer Radiusfunktion stetig konkav verjüngenden Rotationskörper angeordnet. Gemäss der DE-A-3625749 sind solche Instrumente auch zur Gewebeentfernung geeignet und es ist ebenfalls bekannt, durch Einwirkung von Ultraschall biologische Gewebe, insbesondere Knochen zu verbinden.

Bekannt sind Implantate aus sperrschichtbildenden Metallen oder deren Legierungen, deren Oberfläche funktionsspezifische, arteigene Oxidschichten, z. B. Kalziumphosphat mit Bioaktivatoren aufweist (DD 246476). Dies soll ein anwachsen von Gewebe und eine zügige Einheilung gewährleisten.

Nach der DE-A-2333841 ist eine chemische Verbindung mit einer regenerierenden Wirkung auf tierische Gewebe bekannt, die aus einem Manganneoabietat besteht.

Bekannt ist weiterhin ein elektronisches Bioresonanzgerät gemäss EP-A-885628, bei dem ein körpereigenes elektrisches bzw. elektromagnetisches Signal über eine grossflächige Kontaktelektrode empfangen wird und mittels einer Sendevorrichtung wird das zuvor verstärkte Signal dem Körper wieder zugeleitet. Hierdurch soll eine Unterstützung von natürlichen Heilungsprozessen oder eine Verbesserung des Wohlbefindens erreicht werden, ohne dass Fremdstoffe oder Fremdsignale zugeführt werden. Der Abstand zwischen Körper und Sender/Empfänger soll dabei weniger als zwei Meter betragen. Die Signale werden weder örtlich noch gewebespezifisch aufgenommen oder gesendet.

Gemäss DE-A-19858561 kann auch eine Armbanduhr mit einer entsprechenden Zusatzfunktion ausgestattet sein, d. h. Abstrahlung eines elektrischen oder elektromagnetisches Feldes an den menschlichen Körper, um gegen ein störendes Feld zu schützen oder um zu therapieren.

Es ist aber auch aus der Tierwelt bekannt, dass zum Beispiel Katzen in der Lage sind, Vibrationen von 20-140 Hz zu erzeugen, um damit Wunden und Knochenbrüche zu heilen. Besonders wirksam soll ein Schnurren mit Frequenzen von 25-50 Hz sein.

Der Erfindung liegt die Aufgabe zugrunde, eine Einrichtung zur Regenerierung von biologischem Gewebe zu schaffen, die bei unterschiedlichsten biologischen Geweben wie Muskeln, Nerven, Sehnen, Knochen, Knorpel etc. gleichermassen einfach und erfolgversprechend anwendbar ist. Die Aufgabe ist mit den Merkmalen des Patentanspruchs gelöst.

Die Erfindung geht von der Erkenntnis aus, in lebenden Organismen Vorgänge primär durch Wellen bzw. Schwingungen gesteuert werden, sofern sie mit den Schwingungen des Organismus in Resonanz treten. Es kann nur dann eine positive Wirkung erzielt werden, wenn die angebotenen Schwingungen/Spektren den nachgefragten Schwingungen/Spektren entsprechen. Hierbei wird ein breites Spektrum der Benutzerseite für die zu speichernden Bioinformationen berücksichtigt. Mittels Regelung/Feedbackschlaufe und entsprechender Speicherung der Informationen sollen die gespeicherten Bioinformationen individuell resp. spezifisch und automatisch oder manuell eingestellt als optimales Frequenzspektrum abgegeben werden. Dem betreffenden Organismus sollen die für ihn wichtigen Informationen bzgl. Vorbeugung oder Heilung zur Verfügung gestellt werden.

Weiterhin Grundlage für eine Regelung ist, dass sich die elektronisch gespeicherte Bioinformation für jedes Individuum auf das für das individuelle System optimale Frequenzspektrum einstellen lassen muss und zwar möglichst automatisch. Die Informationen für spezifische bzw. generelle Applikationen, die auf entsprechenden Resonanzvorgängen basieren, müssen eingeleitet und gespeichert sein.

Dem betreffenden Organismus sollen die für ihn wichtigen Informationen für eine Heilung, Regenerierung oder zur Prävention durch physikalische Mittel zur Verfügung gestellt werden.

Die Ausführung ermöglicht auch eine Verdichtung/Verstärkung der individuellen Wellen bzw. Schwingungsstruktur, um Gewebeschäden rascher und besser (zum Beispiel ohne bzw. minimale Narbenbildung bei Sehnen, Muskeln oder Nerven) heilen zu können, bzw. das Gewebe zur Heilung zu veranlassen. Im Bereich der Bioinformationen (Spektren, die Körperfunktionen steuern und/oder regeln).

Die Einrichtung enthält mindestens einen Sender/Impulsgeber zur Abgabe spezifischer Frequenzen oder Frequenzspektren, zum Beispiel auch von Ultraschall oder Magnetfelder. Die Form des Impulsgebers ist an diverse Lebewesen bzw. diverse Gewebeformen anpassbar und seine Ansteuerung ist bezüglich der unterschiedlichen Gewebe gleich oder verschieden.

Der Sender/Impulsgeber kann eine einstellbare Leistung abgeben und wird bevorzugt äusserlich auf das betreffende Gewebe bzw. an bestimmte Körperstellen aufgesetzt bzw. möglichst nahe an dieses herangeführt. Einstellbar sind ebenfalls die Leistungszeiten und die Reihenfolge der Leistungsabgabe bei Anwendung mehrerer Impulsgeber. Im Gegensatz zum Stand der Technik kommen auch andere als die körpereigenen Schwingungen zur Anwendung, z.B. ans Speichern oder durch direkte Einleitung

Der Erfindung liegt weiterhin die Aufgabe zugrunde, ein Verfahren zur Regenerierung von solchem biologischen Gewebe zu entwickeln.

Spezifische Bioinformationen werden so zur Verfügung gestellt, dass die Regenerierung des betreffenden Gewebes besser und/oder rascher erfolgt. Messwerte und Feedback des Gewebes auf die Behandlung können gespeichert und ausgewertet werden.

Durch Aufnahme von elektrischen Feldkomponenten und Störfeldkomponenten sind weitere Bioinformationen erhältlich, sofern sie auf solchen Resonanzvorgängen basieren, die eine Optimierung künftiger Behandlungen bzw. Vorbeugung ermöglichen. Die Ausführung erfolgt mit hoher Schwingungsstrukturverdichtung bei Gewebeschäden, um die betreffenden Gewebe zur Heilung zu stimulieren und um weniger Narbenbildung zu erhalten. Die Verdichtung kann eine Multiplikation oder eine Vergrösserung der Spannung beinhalten.

Bei Bedarf kommen noch unterstützende Medikamente und andere physikalische Therapien zur Anwendung.

Die Erfindung kommt bei Muskelverletzungen (Zerrung, Anriss etc.), Nervenverletzungen, Überlastung von Sehnen und Bändern, Gelenkentzündungen oder Verletzungen von Knorpelgewebe, Abnutzungsschädigungen, Allergien, Migräne oder Schmerztherapien usw. zur Anwendung. Aber auch bakterien- oder virenbedingte Schädigungen sind behandelbar. Anwendbar ist die Erfindung ebenso zur (Zell)Regeneration von Organen und des Stoffwechsels und damit zur Gesamtregeneration.
Die Erfindung wird nachfolgend in zwei Ausführungsbeispielen an Hand einer Zeichnung näher beschrieben. Die Zeichnung zeigt in der
- Fig. 1:: den Aufbau der Einrichtung zur Regenerierung von biologischem Gewebe
- Fig. 2:: eine weitere Ausführungsform der Einrichtung.
- Fig. 3:: eine dritte Ausführungsform der Einrichtung.

Ein Impulsgeber, der in seiner Form an verschiedene Gewebe anpassbar ist, enthält mehrere Ansteuerungsstellen 1 bis 21, die als Empfänger/Sender von Bioinformationen dienen (somit ergeben sich bis zu 21 Ansteuerungsstellen) und die mit einem oder mehreren Biospeichern 30 (zum Beispiel bis zu 9 oder mehr) und einem Empfangsteil 40 für die Aufnahme von zusätzlichen Informationsspektren (von bestimmten Körperstellen oder externen Bioinformationen (online) mit gemeinsamer oder individueller Verstärkung/Verdichtung) für ein Feedback verbunden sind. Die Ansteuerungsstellen 1 bis 21 des Impulsgebers sind nach vorgebbaren Reihenfolgen und Zeitintervallen für Bioinformationen einstellbar. Pausenintervalle sind integrierender Bestandteil der Gesamttherapie.

Einsetzbar sind aber zusätzlich beispielweise auch permanente oder wechselnde Magnetfelder oder Ultraschall oder Aqua-Trainer oder Bewegungstherapie. Die Ansteuerungsstellen 1-21 können in einer Einheit oder separat vorgesehen sein. Die Aufnahme und die Einspeisung der erhaltenen bzw. modifizierten Daten erfolgt an der gleichen Stelle.

Ein geschädigtes Gewebe, zum Beispiel eines Beinmuskels 70 wird von den Ansteuerungsstellen 1 bis 21 umgeben, die vom Biospeicher 30 mit einem Wechselfeld in vorgebender Reihenfolge und vorgegebenem Zeitintervall und Leistung beaufschlagt werden. Im Empfangsteil 40 wird das Feedback des Gewebes 70 aufgenommen, ggf. verstärkt und ausgewertet und bei Bedarf nachfolgend die Ansteuerung des Impulsgebers variiert, ggf. auch dessen Frequenzspektrum.

Für einzelne Gewebe kommen individuelle und/oder Standardreihenfolgen zur Anwendung.

Unterstützende Medizinalmittel zur Leitung der Informationen (Kontaktmittel oder via Aufnahmesonde 50) und/oder zur Unterstützung des Regenerierungsprozesses können zur Anwendung gelangen, ebenso andere Therapieformen. Aufgenommene Bioinformationen werden durch spezifische, individuelle oder allgemein gespeicherte Bioinformationen ergänzt, die ihrerseits verstärkt, verdichtet oder multipliziert wurden, bevor sie (wieder) an das geschädigte Gewebe abgegeben werden.

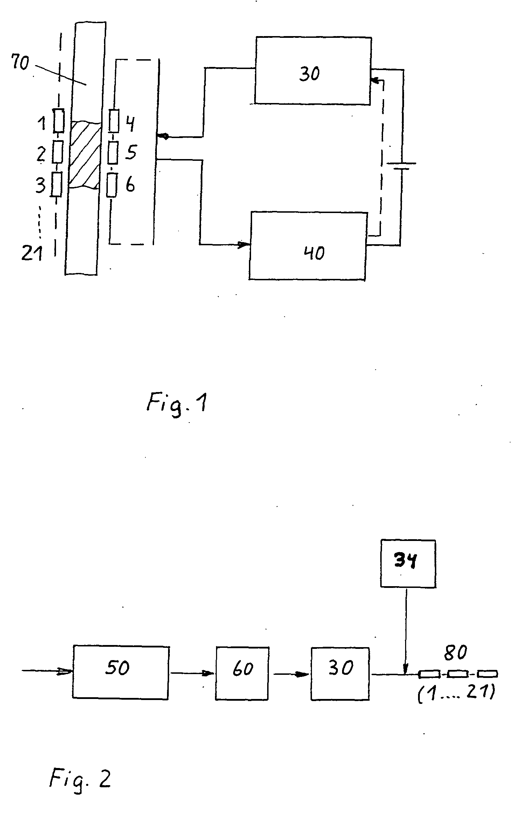

In einer weiteren Ausführungsform (Fig. 2) ist mindestens eine Aufnahmesonde 50 dicht an einem biologischen Gewebe angeordnet, zwecks Aufnahme/Empfang einer gesunden Strukturinformation (Bioresonanz nur, wenn Sender und Empfänger an gleicher Stelle oder übereinander angeordnet sind) aus einer vorbestimmten Region. Wie auch bei der ersten Ausführungsform muss zunächst die Schwingungsstruktur an unverletzter Stelle ermittelt werden, um die Einrichtung so einstellen zu können, dass die gesamte Schwingungsstruktur übertragbar ist.

Diese Informationen gelangen mittels üblicher Datenübertragung auf einen Verstärker 60, wo sie bis zu 50-fach verstärkt/verdichtet (Verdichtung = Anzahl der Sendeimpulse) werden. Entsprechende Einstellungen und Vorgabe der Wichtigkeit von Verstärkungsfaktoren sind vorzunehmen bzw. das System soll selbständig die notwendigen Verstärkungen vornehmen.

Die verstärkten Bioinformationen gelangen in eine oder mehrere, eingeschaltete Senderspulen 80 (Ansteuerungsstelle) und damit einem zu heilenden Gewebe zugeführt werden. Zusätzlich können aus 1-3 weiteren Biospeichern (Verstärker 34) zusätzliche und ggf. verstärkte/verdichtete (Faktor 1-50x) Bioinformationen dem Sender zugeführt werden. Hierbei handelt es sich um individuelle Bioinformationen zur Regulierung bzw. Anregung des Regenerierungsprozesses. Die Einstellung einer optimalen Verstärkung erfolgt entweder über Messungen am betreffenden Körper oder dem Körper werden die Bioinformationen so zur Verfügung gestellt, dass die Einrichtung daraus selbst eine optimale Verdichtung bzw. Verstärkung ermitteln bzw. auswählen kann.

Die gesamten Bioinformationen werden vom Sender 80 auf das Gewebe übertragen. Aufnahme und Einspeisung von Bioinformationen erfolgen an verschiedenen Stellen. Es ist wichtig, zunächst die Schwingungsstruktur und den Verdichtungsfaktor zu bestimmen, der eingesetzt werden muss, bis sich diese Struktur regeneriert. Der Verdichtungsfaktor kann von Struktur (Gewebe) zu Struktur verschieden sein. Die Aufnahme der unverletzten Struktur muss an einer von der Einspeisung abweichenden Stelle erfolgen, da die aufgenommene Information im Original vorhanden sein muss. Sie ist die Basis einer Verdichtung, die Verdichtung wiederum Basis einer Geweberegenerierung.

Die "gesunde" Bioinformation wird zur Heilung des geschädigten Gewebes am optimalen Ort geprägt. Eine dauerhafte Prägung kann nur entstehen, wenn die notwendige Verdichtung (= Druck) gewährleistet ist.

Senderspulen 80 resp. Ansteuerungsstellen 1-21 können stationär bzw. flexibel und damit wand- bzw. mattenförmig ausgebildet sein. Dabei können die einzelnen Ansteuerungsstellen in Linien bzw. Streifen angeordnet sein, dies wiederum gleichmässig beabstandet oder abwechselnd mit unterschiedlichen Abständen (einzeln oder in Gruppen) zu einer Bezugsfläche oder auch netzartig/maschenförmig. Von derartigen Biofeldern kann ein Körper "durchflutet" werden, Sender- bzw. Aufnehmermatten können z. B. 3x7 Spulen mit bestimmten Aussendurchmessern und Drahtstärken enthalten. Die Leiter können gerade und senkrecht oder geneigt dazu angeordnet sein, wobei jeder dritte Draht zusammengefasst wird.

Je nach Einsatzzweck können verschiedene Programme bzgl. Dauer, Intervall, Häufigkeit und/oder Intensität zur Anwendung kommen. Bei Bedarf auch in Kombination mit anderen Mitteln, wie Gleich- oder Wechselfelder, Ultraschall, Regenerationspausen etc.

Nach bestimmten bzw. individuell ausgetesteten Zeitverhältnissen können analog zur ersten Ausführungsform zusätzlich auch Medikamente zur Anwendung gelangen.

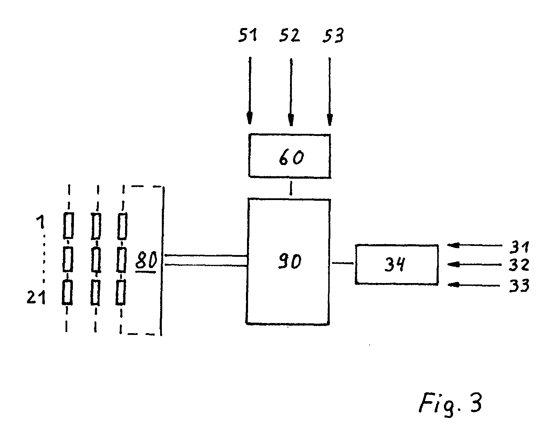

Mit einer weiteren Ausführungsform (Fig. 3) der Einrichtung, die insbesondere für den mobilen Einsatz geeignet ist, wird für einen feinstofflichen Test die Information aus dem Speicher 31-33 bezogen/gesendet. Zusätzlich oder alternativ können auf Informationen aus den Aufnahmesonden 51-53 bezogen werden. Die Aufnahmesonden 51-53 sind einzeln zuschaltbar. Mit Einschaltung der Senderspulen 80 der Aussteuerungsstellen 1-21 werden weitere Informationen bezogen. Nach Einschalten des Gerätes 90 (Verstärker 60) wird anhand der bezogenen Informationen eine entsprechende Verstärkung/-Verdichtung für die Informationen des Biospeichers 30 und der Bioinformationen aus den Aufnahmesonden 51-53 eingestellt. Über die Ansteuerungsstellen 1-21 gelangen die Informationen in das Gewebe 70. Anschliessend wird das Gerät 90 (Auswertungseinheit) bis zum Ablauf einer optimalen Zeit betrieben und abgeschaltet. Dieser Vorgang wird mit veränderten/neuen Einstellungen bis zu 6x wiederholt.

Über die Aufnahmesonden 51-53 können weiterhin spezifische Informationen von Heilmitteln/Medikamenten direkt eingegeben werden.

Bioresonanz entsteht, wenn die Aufnahmesonden 51-53 auf die Senderspulen 80 gelegt werden.

Der Verstärker 60 dient der VerstärkungNerdichtung der an bestimmten Stellen des Individuums (70) oder extern (online) aufgenommener Bioinformationen.

Die genannten Ausführungsformen sind kombinierbar, da lediglich die Aufnahme der Bioinformationen an einer anderen Stelle als die Einspeisung erfolgt.

### Bezugszeichen

- 1-21: Ansteuerungsstellen
- 30-33: Biospeicher
- 34: Verstärker für Biospeicher
- 40: Empfangsteil/Steuerteil
- 50-53: Aufnahmesonde
- 60: Verstärker für Aufnahmesonde
- 70: Gewebe
- 80: Senderspule
- 90: Gerät

## Patentansprüche

1. Einrichtung zur Regenerierung von biologischem Gewebe, insbesondere bei Menschen und Tieren, umfassend mindestens einen Sender von Bioinformationen, einen Biospeicher (30-33) resp. eine Auswerteeinheit für aufgenommene bzw. zu sendende Bioinformationen.

2. Einrichtung nach Anspruch 1, **dadurch gekennzeichnet, dass** zwischen Empfänger und Biospeicher (30-33) ein Verstärker (60) zur Verstärkung bzw. Verdichtung dieser Bioinformationen vorgesehen ist.

3. Einrichtung nach Anspruch 1 oder 2, **dadurch gekennzeichnet, dass** der Empfänger als Aufnahmesonde (50-53) oder als mindestens eine Ansteuerungsstelle (1-21) ausgebildet ist.

4. Einrichtung nach Anspruch 3, **dadurch gekennzeichnet, dass** bis zu 21 Ansteuerungsstellen (1-21) vorgesehen sind, die Bioinformationen auf den Körper senden, bzw. in den Körper einspeisen.

5. Einrichtung nach mindestens einem der Ansprüche 1 bis 4, **dadurch gekennzeichnet, dass** ein weiterer Biospeicher (34) vorgesehen ist.

6. Einrichtung nach mindestens einem der Ansprüche 1 bis 3 oder 5, **dadurch gekennzeichnet, dass** der Sender (80) zur Abgabe von verdichteten Bioinformationen vorgesehen ist.

7. Einrichtung nach mindestens einem der Ansprüche 1 bis 6, **dadurch gekennzeichnet, dass** Empfänger und Sender an verschiedenen Gewebestellen angelegt sind.

8. Verfahren zur Regenerierung von biologischem Gewebe, insbesondere von Menschen und Tieren, umfassend die Schritte
a, Anlegen mindestens eines Empfängers von Bioinformationen an ein gesundes biologisches Gewebe bzw. an eine entsprechende Körperstelle und/oder von aussen
b, Verstärkung dieser Bioinformationen
c, Abgabe von verstärkten Bioinformationen an das zu regenerierende biologische Gewebe.

9. Verfahren nach Anspruch 8, **dadurch gekennzeichnet, dass** unterstützend weitere Therapien/Medikationen einwirken.

10. Verfahren nach Anspruch 8 oder 9, **dadurch gekennzeichnet, dass** die aufgenommenen und/oder die abgegebenen Bioinformationen verstärkt und/oder verdichtet oder multipliziert werden.

11. Verfahren nach Anspruch 8 bis 10, **dadurch gekennzeichnet, dass** Aufnahme und Abgabe der Bioinformationen an gleichen (Bioresonanz) oder an unterschiedlichen Gewebestellen erfolgen.

12. Vorrichtung nach einem der Ansprüche 1 bis 7, **dadurch gekennzeichnet, dass** Senderspulen (80) stationär/wandartig oder flexibel/mattenartig ausgebildet sind
